# EUROPEAN PATENT APPLICATION

(11) **EP 4 822 582 A1**
(43) Date of publication of application: **14.10.2026**
(21) Application number: 25913438.5
(22) Date of filing: 05.12.2025
(51) Int. Cl.: G06F 30/20, H01M 4/62, H01M 4/02, G16C 60/00, G06F 111/04, G06F 111/18

(54) **METHOD AND SYSTEM FOR DESIGNING THREE-DIMENSIONAL ELECTRODE STRUCTURE**

(30) Priority: 23.12.2024 KR 20240193889
(71) Applicant: LG Energy Solution, Ltd., Seoul 07335 (KR)
(72) Inventor: PARK, Joonam, Daejeon 34122 (KR); PARK, Sun Ho, Daejeon 34122 (KR); KIM, Eun Bee, Daejeon 34122 (KR); BAE, Kyungtaek, Daejeon 34122 (KR); KIM, Hyeongseok, Daejeon 34122 (KR); LEE, Bora, Daejeon 34122 (KR)
(74) Representative: Goddar, Heinz J.
(86) International application number: PCT/KR2025/020840
(87) International publication number: WO 2026/142048

(57) **Abstract**

A three-dimensional electrode structure design system according to an embodiment of the present invention may comprise: a structure forming module configured to form a conductive additive and binder (CBD) structure using a first design parameter input for the CBD structure, and form an electrode structure using a second design parameter input for the electrode structure; a calculation module configured to calculate an effective electrical conductivity of the CBD structure using an intrinsic electrical conductivity of a conductive additive and an intrinsic electrical conductivity of a binder, calculate an effective electrical conductivity of the electrode structure using an intrinsic electrical conductivity of the active material, an intrinsic electrical conductivity of CBD particles, and an intrinsic electrical conductivity of the current collector, and calculate a calculated resistivity of the electrode structure by converting the effective electrical conductivity of the electrode structure into a reciprocal; a correction module configured to calculate a deviation by comparing the calculated resistivity with a target resistivity and correct a variable or shape of an electrode structure in a direction of minimizing the deviation; and a backtracking module configured to calculate the intrinsic electrical conductivity of the active material, the intrinsic electrical conductivity of CBD particles, the intrinsic electrical conductivity of the current collector, and a third design parameter, all of which are included in the corrected electrode structure.

## Description

### [Technical Field]

### Cross-reference to related application(s)

This application claims the benefit of priority based on Korean patent application No. 10-2024-0193889 filed on December 23, 2024, and the entire contents disclosed in the document of the corresponding Korean patent application are incorporated as a part of the present specification.

The present invention relates to a method and system for designing a three-dimensional electrode structure.

### [Background Art]

Digital twin technology is a technology for realizing objects in reality in a virtual space within a computer to simulate various situations that may occur in reality and to predict the results. Such digital twin technology may be utilized for research and development of a secondary battery. That is, the cost and time used in an actual secondary battery process may be reduced by modeling a three-dimensional electrode structure of a secondary battery in a virtual space within a computer through the digital twin technology and verifying the characteristics of the three-dimensional electrode structure.

Meanwhile, since a three-dimensional electrode structure formed by the conventional digital twin technology is at a micro-size level, it is difficult to individually distinguish a nano-sized conductive additive and a binder within the three-dimensional electrode structure. Due to this, there is a limit in accurately predicting the resistivity of a three-dimensional electrode structure according to shapes of the conductive additive and the binder.

### [Disclosure]

### [Technical Problem]

An object of the present invention is to provide a method and system for designing a three-dimensional electrode structure that may form a three-dimensional electrode structure and calculate the resistivity of the three-dimensional electrode structure according to the shapes of a conductive additive and a binder included therein.

### [Technical Solution]

A three-dimensional electrode structure design system according to an embodiment of the present invention may comprise: a structure forming module configured to form a conductive additive and binder (CBD) structure using a first design parameter input for the CBD structure, and form an electrode structure using a second design parameter input for the electrode structure; a calculation module configured to calculate an effective electrical conductivity of the CBD structure using an intrinsic electrical conductivity of a conductive additive and an intrinsic electrical conductivity of a binder, calculate an effective electrical conductivity of the electrode structure using an intrinsic electrical conductivity of the active material, an intrinsic electrical conductivity of CBD particles, and an intrinsic electrical conductivity of the current collector, and calculate a calculated resistivity of the electrode structure by converting the effective electrical conductivity of the electrode structure into a reciprocal; a correction module configured to calculate a deviation by comparing the calculated resistivity with a target resistivity and correct a variable or shape of an electrode structure in a direction of minimizing the deviation; and a backtracking module configured to calculate the intrinsic electrical conductivity of the active material, the intrinsic electrical conductivity of CBD particles, the intrinsic electrical conductivity of the current collector, and a third design parameter, all of which are included in the corrected electrode structure.

The structure forming module may comprise a conductive additive and binder structure forming module configured to set the domain and voxel sizes based on the first design parameter and form a conductive additive in the domain using the first design parameter.

The structure forming module may comprise an electrode structure formation module configured to set the domain and voxel sizes based on the second design parameter and form an active material, CBD particles, and a current collector in the domain using the second design parameter.

The calculation module may comprise an effective electrical conductivity calculation module configured to calculate the effective electrical conductivity of the CBD structure based on the Laplace equation and the Ohm's Law.

The calculation module may comprise a resistivity calculation module configured to calculate the effective electrical conductivity of the electrode structure by substituting the effective electrical conductivity of the CBD structure for the intrinsic electrical conductivity of CBD particles.

The correction module may calculate a deviation between the calculated resistivity and the target resistivity, may perform correction for the electrode structure when the deviation is greater than or equal to a predetermined reference value, and may not perform correction for the electrode structure when the deviation is less than the predetermined reference value.

The correction module may, when the deviation between the calculated resistivity and the target resistivity is greater than or equal to the predetermined reference value, compare magnitudes of the calculated resistivity and the target resistivity, and when the target resistivity is smaller than the calculated resistivity, correct the electrode structure by increasing the intrinsic electrical conductivity of the active material, the intrinsic electrical conductivity of CBD particles, or the intrinsic electrical conductivity of the current collector.

The correction module may, when the deviation between the calculated resistivity and the target resistivity is greater than or equal to the predetermined reference value, compare magnitudes of the calculated resistivity and the target resistivity, and when the target resistivity is larger than the calculated resistivity, correct the electrode structure by decreasing the intrinsic electrical conductivity of the active material, the intrinsic electrical conductivity of CBD particles, or the intrinsic electrical conductivity of the current collector.

The correction module may, when the deviation between the calculated resistivity and the target resistivity is greater than or equal to the predetermined reference value, compare magnitudes of the calculated resistivity and the target resistivity, and when the target resistivity is smaller than the calculated resistivity, correct the electrode structure by dispersing CBD particles in the domain of the electrode structure.

The correction module may, when the deviation between the calculated resistivity and the target resistivity is greater than or equal to the predetermined reference value, compare magnitudes of the calculated resistivity and the target resistivity, and when the target resistivity is larger than the calculated resistivity, correct the electrode structure by agglomerating CBD particles in the domain of the electrode structure.

The correction module may, when a volume deviation between CBD particles in the corrected electrode structure and CBD particles in the electrode structure before the shape is corrected is greater than or equal to a predetermined reference value, additionally form CBD particles in a domain of the electrode structure.

The correction module may calculate the calculated resistivity of the corrected electrode structure and determine whether it is necessary to repeat the correction for the electrode structure based on whether a deviation between the calculated resistivity of the corrected electrode structure and the target resistivity is greater than or equal to a predetermined value.

A three-dimensional electrode structure design method according to an embodiment of the present invention may comprise: forming, by a structure forming module, a conductive additive and binder (CBD) structure using a first design parameter input for the CBD structure, and calculating, by a calculation module, an effective electrical conductivity of the CBD structure using an intrinsic electrical conductivity of a conductive additive and an intrinsic electrical conductivity of a binder; forming, by the structure forming module, an electrode structure using a second design parameter input for the electrode structure, calculating, by the calculation module, an effective electrical conductivity of the electrode structure using an intrinsic electrical conductivity of the active material, an intrinsic electrical conductivity of CBD particles, and an intrinsic electrical conductivity of the current collector, and calculating a calculated resistivity of the electrode structure by converting the effective electrical conductivity of the electrode structure into a reciprocal; calculating, by a correction module, a deviation by comparing the calculated resistivity with a target resistivity, and correcting a variable or shape of an electrode structure in a direction of minimizing the deviation; and calculating, by a backtracking module, the intrinsic electrical conductivity of the active material, the intrinsic electrical conductivity of CBD particles, the intrinsic electrical conductivity of the current collector, and a third design parameter, all of which are included in the corrected electrode structure.

The step of calculating the effective electrical conductivity of the CBD structure may comprise: setting, by the structure forming module, domain and voxel sizes based on the first design parameter; and forming a conductive additive in a domain using the first design parameter.

The step of calculating the calculated resistivity of the electrode structure may comprise: setting, by the structure forming module, the domain and voxel sizes based on the second design parameter; and forming an active material, CBD particles, and a current collector in a domain using the second design parameter.

The step of calculating the calculated resistivity of the electrode structure may comprise: calculating, by the calculation module, the effective electrical conductivity of the electrode structure by substituting the effective electrical conductivity of the CBD structure for the intrinsic electrical conductivity of CBD particles.

The step of correcting the variable or shape of an electrode structure may comprise: calculating, by the correction module, the deviation between the calculated resistivity and the target resistivity; and performing correction for the electrode structure when the deviation between the calculated resistivity and the target resistivity is greater than or equal to a predetermined reference value, and not performing correction for the electrode structure when the deviation between the calculated resistivity and the target resistivity is less than the predetermined reference value.

The step of correcting the variable or shape of an electrode structure may comprise: calculating a deviation between the calculated resistivity and the target resistivity and determining whether the deviation between the calculated resistivity and the target resistivity is greater than or equal to a predetermined reference value; when the deviation between the calculated resistivity and the target resistivity is greater than or equal to the predetermined reference value, comparing magnitudes of the calculated resistivity and the target resistivity; when the target resistivity is smaller than the calculated resistivity, correcting the electrode structure by increasing the intrinsic electrical conductivity of the active material, the intrinsic electrical conductivity of CBD particles, or the intrinsic electrical conductivity of the current collector; when the target resistivity is larger than the calculated resistivity, correcting the electrode structure by decreasing the intrinsic electrical conductivity of the active material, the intrinsic electrical conductivity of CBD particles, or the intrinsic electrical conductivity of the current collector; and calculating the calculated resistivity of the corrected electrode structure.

The step of correcting the variable or shape of an electrode structure comprises: calculating a deviation between the calculated resistivity and the target resistivity and determining whether the deviation between the calculated resistivity and the target resistivity is greater than or equal to a predetermined reference value; when the deviation between the calculated resistivity and the target resistivity is greater than or equal to the predetermined reference value, comparing magnitudes of the calculated resistivity and the target resistivity; when the target resistivity is smaller than the calculated resistivity, correcting the electrode structure by dispersing CBD particles in the domain of the electrode structure; and when the target resistivity is larger than the calculated resistivity, correcting the electrode structure by agglomerating CBD particles in the domain of the electrode structure.

The step of correcting the variable or shape of an electrode structure may comprise: determining whether a volume deviation between CBD particles in the corrected electrode structure and CBD particles in the electrode structure before correction is less than a predetermined reference value; additionally forming CBD particles in the domain of the electrode structure when the volume deviation between the CBD particles in the corrected electrode structure and the CBD particles in the electrode structure before correction is greater than or equal to the predetermined reference value; and calculating the calculated resistivity of the corrected electrode structure when the volume deviation between the CBD particles in the corrected electrode structure and the CBD particles in the electrode structure before correction is less than the predetermined reference value.

### [Advantageous Effects]

According to an embodiment of the present invention, the resistivity of a three-dimensional electrode structure may be calculated according to the shapes of a conductive additive and a binder included in the three-dimensional electrode structure.

In addition, according to an embodiment of the present invention, intrinsic physical properties or a design parameter of a three-dimensional electrode structure having a target resistivity, or shape information of a conductive additive and a binder thereof may be obtained.

Effects obtainable in the present disclosure are not limited to the effects mentioned above, and other effects not mentioned may be clearly understood by those of ordinary skill in the art to which the present disclosure belongs from the following description.

### [Description of the Drawings]

FIG. 1 is a block diagram of a three-dimensional electrode structure design system according to an embodiment of the present invention.
FIGS. 2 to 4 are an example of a CBD structure formed by a CBD structure forming module according to an embodiment of the present invention.
FIGS. 5a and 5b are examples of electrode structures formed by the electrode structure formation module according to an embodiment of the present invention.
FIG. 6 is a graph showing the effective electrical conductivity of the CBD structure according to the content ratio of conductive additive to binder.
FIG. 7 shows a process in which the correction module corrects the shape of the electrode structure using the shape tracking algorithm according to an embodiment of the present invention.
FIG. 8 is a flowchart of the three-dimensional electrode structure design method according to an embodiment of the present invention.
FIG. 9 is a flowchart illustrating a step of correcting the electrode structure according to an embodiment of the present invention.
FIG. 10 is a flowchart of the step of correcting the electrode structure according to another embodiment of the present invention.

### [Mode for Invention]

In describing the embodiments disclosed in the present specification, a detailed description of related known technology is omitted when it is determined that it may obscure the gist of the embodiments disclosed in the present specification. In addition, the accompanying drawings are only for easily understanding the embodiments disclosed in the present specification, and the technical spirit disclosed in the present specification is not limited by the accompanying drawings, and it should be understood to include all changes, equivalents, or substitutes included in the spirit and technical scope of the present invention.

Terms including ordinal numbers such as first, second, etc., may be used to describe various components, but the components are not limited by the terms. The terms are used only for the purpose of distinguishing one component from another component.

When it is mentioned that a certain component is "connected" or "coupled" to another component, it should be understood that it may be directly connected or coupled to the other component, but another component may exist in between. On the other hand, when it is mentioned that a certain component is "directly connected" or "directly coupled" to another component, it should be understood that no other component exists in between.

In the present application, terms such as "comprise" or "have" are intended to designate the existence of features, numbers, steps, operations, components, parts, or combinations thereof described in the specification, and it should be understood that it does not preclude the possibility of existence or addition of one or more other features, numbers, steps, operations, components, parts, or combinations thereof in advance.

Hereinafter, the present invention will be described in detail with reference to the accompanying drawings.

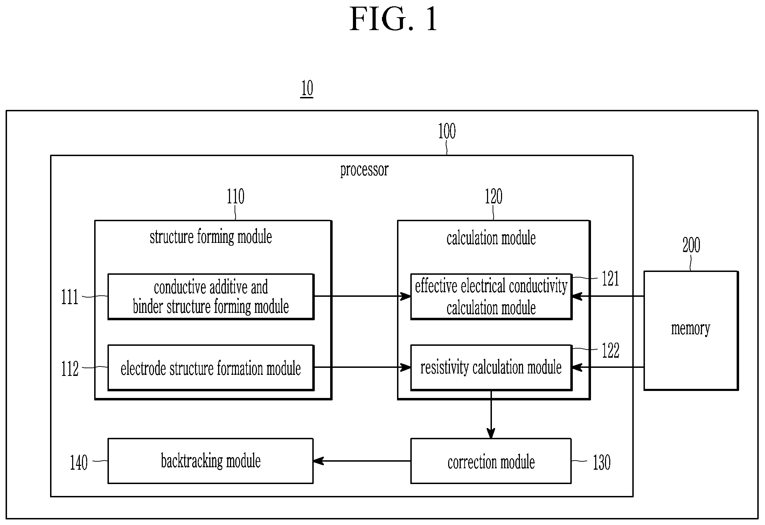

FIG. 1 is a block diagram of a three-dimensional electrode structure design system 10 according to an embodiment of the present invention.

Referring to FIG. 1, a three-dimensional electrode structure design system 10 according to an embodiment of the present invention may comprise a processor 100 and a memory 200.

The processor 100 may form a three-dimensional structure, and may calculate an effective electrical conductivity and a resistivity representing electrical characteristics of the formed three-dimensional structure. The processor 100 may correct the three-dimensional structure using the effective electrical conductivity and the resistivity of the three-dimensional structure. According to an embodiment, the processor 100 may comprise a structure forming module 110, a calculation module 120, a correction module 130, and a backtracking module 140.

The structure forming module 110 may form a three-dimensional structure using a design parameter input from the outside. Here, the outside may be a user terminal, a higher-level controller, or the like. According to an embodiment, the structure forming module 110 may comprise a conductive additive and binder structure forming module 111 and an electrode structure formation module 112.

The conductive additive and binder structure forming module 111 may form a three-dimensional structure of a conductive additive and binder (hereinafter, referred to as a 'CBD structure'). The CBD structure forming module 111 may set the domain and voxel sizes based on a first design parameter input for the CBD structure. The CBD structure forming module 111 may form a conductive additive in a domain using the first design parameter. At this time, a part other than the part corresponding to the conductive additive in the domain corresponds to binder.

Here, the first design parameter means a variable required to form the CBD structure, and may include true densities of the conductive additive and the binder, a content ratio of conductive additive and binder, a shape and diameter of conductive additive particles, a curl-index, and the like.

According to an embodiment, the CBD structure forming module 111 may set the size of the domain based on the shape and diameter of conductive additive particles, and may set the size of the voxel based on the size of the domain. For example, the CBD structure forming module 111 may set a length of each side of the domain to a value greater than or equal to N times (where N is a natural number of 2 or more) the diameter of the conductive additive particles. In addition, the size of the voxel may be set to 1/10^{N} (where N is a natural number of 2 or more) based on the size of the domain. At this time, the domain and voxel sizes set by the CBD structure forming module 111 may be in nano units. However, the domain and voxel sizes set by the CBD structure forming module 111 are not limited thereto, and may be freely set as needed. According to an embodiment, the size of domain and voxel may be set by a user and input through the processor 100 as the first design parameter.

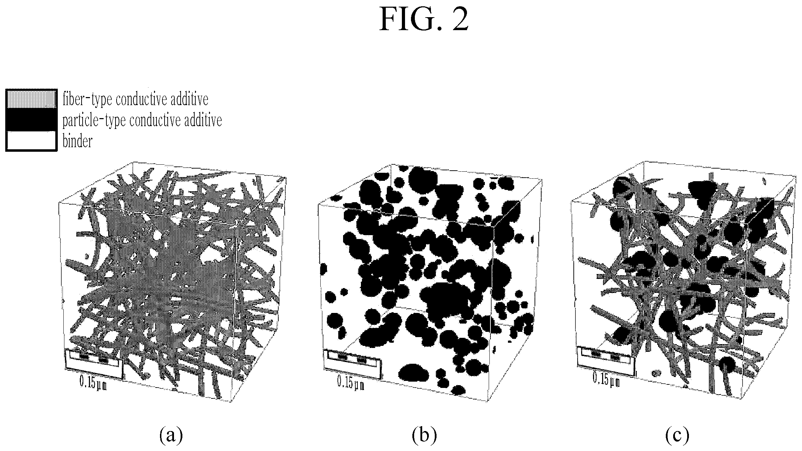

FIGS. 2 to 4 are an example of a CBD structure formed by the CBD structure forming module 111 according to an embodiment of the present invention.

FIG. 2(a) is an example of a CBD structure including a fiber-type conductive additive, FIG. 2(b) is an example of a CBD structure including a particle-type conductive additive, and FIG. 2(c) is an example of a CBD structure in which fiber-type and particle-type conductive additives are mixed. Referring to FIGS. 2(a) to 2(c), conductive additives having various shapes and diameters may be included in the CBD structure formed by the CBD structure forming module 111 according to an embodiment of the present invention.

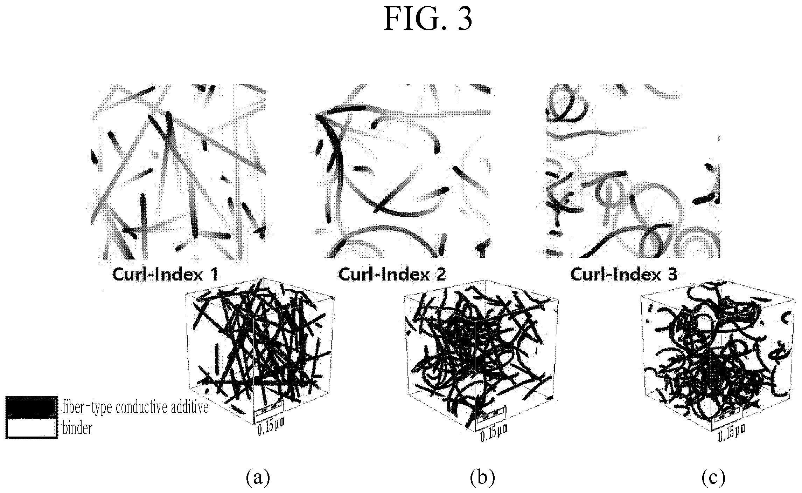

FIGS. 3(a) to 3(c) are examples of CBD structures including a fiber-type conductive additive, and are examples of CBD structures in which the true density of the conductive additive and binder, the content ratio of conductive additive to binder, and the diameter of the fiber-type conductive additive among the first design parameters are the same, but having different curl-indices. Referring to FIGS. 3(a) to 3(c), it may be seen that the shape of the conductive additive formed in the domain by the CBD structure forming module 111 varies according to the curl-index included in the first design parameter.

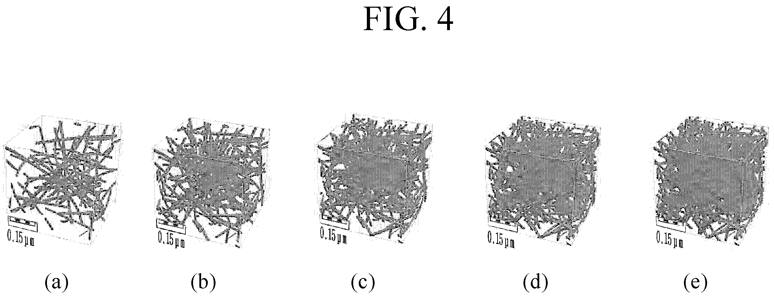

FIGS. 4(a) to 4(e) are examples of CBD structures including a fiber-type conductive additive, and are examples of CBD structures in which the content ratio of conductive additive to binder among the first design parameters is different from each other. FIG. 4(a) is an example of a CBD structure in which the content ratio of conductive additive to binder is 0.2 wt%, FIG. 4(b) is an example of a CBD structure in which the content ratio of conductive additive to binder is 0.4 wt%, FIG. 4(c) is an example of a CBD structure in which the content ratio of conductive additive to binder is 0.6 wt%, FIG. 4(d) is an example of a CBD structure in which the content ratio of conductive additive to binder is 0.8 wt%, and FIG. 4(e) is an example of a CBD structure in which the content ratio of conductive additive to binder is 1.0 wt%. Referring to FIGS. 4(a) to 4(e), it may be seen that the volume ratio of the conductive additive formed in the domain by the CBD structure forming module 111 varies according to the content ratio of conductive additive to binder included in the first design parameter.

The electrode structure formation module 112 may form a three-dimensional structure for an electrode (hereinafter, referred to as an 'electrode structure'). The electrode structure formation module 112 may set the domain and voxel sizes based on a second design parameter input for the electrode structure. The electrode structure formation module 112 may form an active material, conductive additive and binder (CBD) particles, and a current collector in a domain using the second design parameter.

Here, the second design parameter means a variable required to form the electrode structure, and may include content and true density of each component included in the electrode structure, size distribution and sphericity of active material, content ratio of conductive additive to binder, CBD dispersion degree, current collector thickness, and the like.

According to an embodiment, the electrode structure formation module 112 may set the domain and voxel sizes based on the diameter of the active material particles. For example, the electrode structure formation module 112 may set the size of the domain to N times (where N is a natural number of 2 or more) based on the diameter of the largest active material particle, and the size of the voxel to 1/M times (where M is a natural number of 2 or more) based on the diameter of the smallest active material particle. At this time, the domain and voxel sizes set by the electrode structure formation module 112 may be in micro units. However, the domain and voxel sizes set by the electrode structure formation module 112 are not limited thereto, and may be freely set as needed. According to an embodiment, the sizes of the domain and the voxel may be set by a user and input through the processor 100 as the second design parameter.

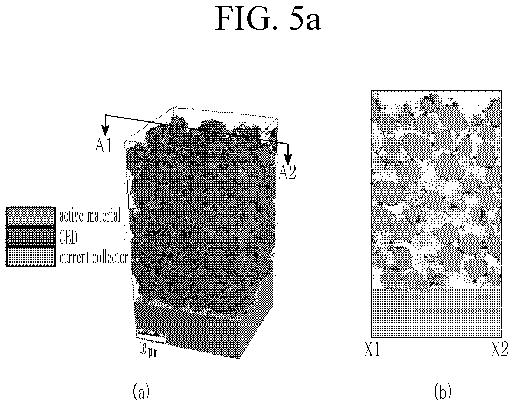

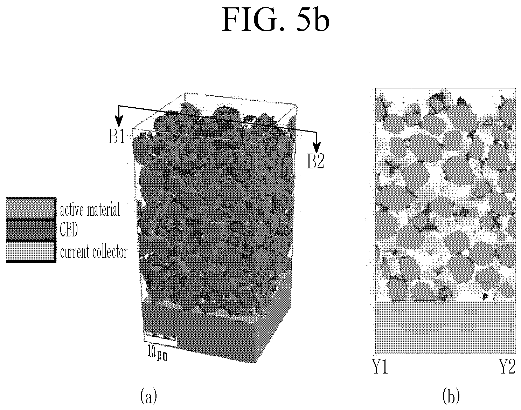

FIGS. 5a and 5b are examples of electrode structures formed by the electrode structure formation module 112 according to an embodiment of the present invention.

FIG. 5a(a) is an example of an electrode structure that is formed by an electrode structure when the true density of the conductive additive is 2 g/cm³, the true density of the binder is 1.78 g/cm³, the content ratio of conductive additive to binder is 1:5 wt%, and the CBD dispersion degree is 0.04071. FIG. 5a(b) is a cross-section taken along A1-A2 in FIG. 5a(a).

FIG. 5b(a) is an example of an electrode structure formed when the true density of the conductive additive is 2 g/cm³, the true density of the binder is 1.78 g/cm³, the content ratio of conductive additive to binder is 1:5 wt%, and the CBD dispersion degree is 0.07265. FIG. 5b(b) is a cross-section taken along B1-B2 in FIG. 5b(a).

Referring to FIGS. 5a to 5b, it may be seen that the shape of the electrode structure formed in the domain by the electrode structure formation module 112 varies according to the CBD dispersion degree included in the second design parameter.

The calculation module 120 may calculate an effective electrical conductivity or a resistivity of the three-dimensional structure formed by the structure forming module 110. According to an embodiment, the calculation module 120 may comprise an effective electrical conductivity calculation module 121 and a resistivity calculation module 122.

The effective electrical conductivity calculation module 121 may calculate the effective electrical conductivity of the CBD structure. The effective electrical conductivity calculation module 121 may calculate the effective electrical conductivity of the CBD structure using the intrinsic electrical conductivity of the conductive additive and the intrinsic electrical conductivity of the binder. Here, the intrinsic electrical conductivity of the conductive additive and the intrinsic electrical conductivity of the binder may be stored in the memory 200. The effective electrical conductivity of the CBD structure calculated by the effective electrical conductivity calculation module 121 may be calculated differently according to the shape and size of the conductive additive and the content ratio of conductive additive to binder in the CBD structure.

At this time, various conventionally known methods may be applied for calculating the effective electrical conductivity of the CBD structure using the intrinsic electrical conductivity of the conductive additive and the intrinsic electrical conductivity of the binder. According to an embodiment, the effective electrical conductivity calculation module 121 may calculate the effective electrical conductivity of the CBD structure based on the Laplace equation and the Ohm's Law. For example, the effective electrical conductivity calculation module 121 may perform numerical analysis on the CBD structure using the Laplace equation, may obtain an average current density and an average electric field of the CBD structure by simulating a situation in which a current flows through the CBD structure, and may calculate the effective electrical conductivity by dividing the average current density by the average electric field.

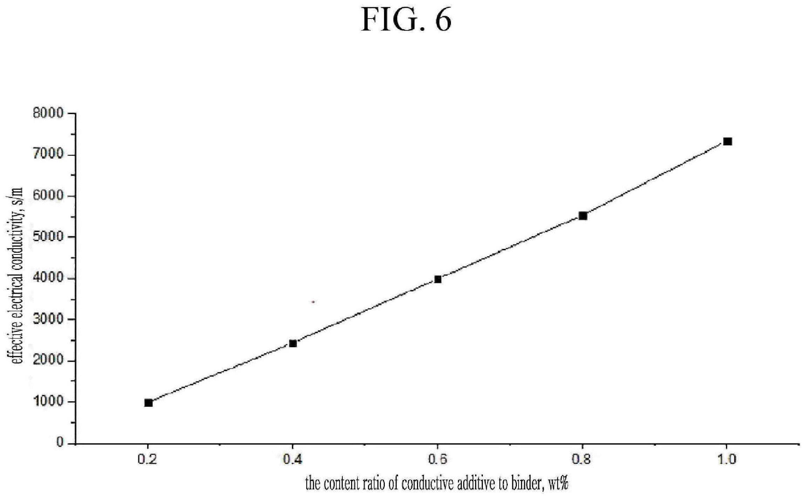

FIG. 6 is a graph showing the effective electrical conductivity of the CBD structure according to the content ratio of conductive additive to binder.

FIG. 6 shows the effective electrical conductivity calculated by the effective electrical conductivity calculation module 121 according to an embodiment of the present invention for the CBD structures of FIG. 4. Referring to FIG. 6, it may be seen that the content ratio of conductive additive to binder and the effective electrical conductivity of the CBD structure are in a proportional relationship with each other.

The resistivity calculation module 122 may calculate a calculated resistivity of the electrode structure. The resistivity calculation module 122 may calculate an effective electrical conductivity of the electrode structure using an intrinsic electrical conductivity of the active material, an intrinsic electrical conductivity of CBD particles, and an intrinsic electrical conductivity of the current collector.

Here, the intrinsic electrical conductivity of the active material and the intrinsic electrical conductivity of the current collector may be stored in the memory 200. The resistivity calculation module 122 may use the effective electrical conductivity of the CBD structure received from the effective electrical conductivity calculation module 121 as the intrinsic electrical conductivity of CBD particles. That is, the resistivity calculation module 122 may calculate the calculated resistivity of the electrode structure by using the effective electrical conductivity of the CBD structure calculated by the effective electrical conductivity calculation module 121 as the intrinsic electrical conductivity of CBD particles formed in the domain of the electrode structure by the electrode structure formation module 112.

Since the electrode structure is of a micro size, nano-sized conductive additive and binder maynot be individually distinguished within the electrode structure. Due to this, the influence according to the shape characteristics of the conductive additive and binder is not reflected in the electrical characteristics obtained from the electrode structure. On the other hand, when calculating the electrical characteristics (i.e., resistivity) of the electrode structure, the resistivity calculation module 122 according to an embodiment of the present invention calculates the electrical characteristics of the CBD structure in which the influence according to the shape characteristics of the conductive additive and binder is reflected, and obtains the electrical characteristics of the electrode structure using the same, so that the electrical characteristics of the electrode structure in which the influence according to the shape characteristics of the conductive additive and binder is reflected may be obtained.

At this time, various conventionally known methods may be applied to a method of calculating the effective electrical conductivity of the electrode structure using the intrinsic electrical conductivity of the active material, the intrinsic electrical conductivity of CBD particles, and the intrinsic electrical conductivity of the current collector. According to an embodiment, the resistivity calculation module 122 may calculate the effective electrical conductivity of the electrode structure based on the Laplace equation and the Ohm's Law. For example, the resistivity calculation module 122 may perform numerical analysis on the electrode structure using the Laplace equation, may obtain an average current density and an average electric field of the electrode structure by simulating a situation in which a current flows through the electrode structure, and may calculate the effective electrical conductivity by dividing the average current density by the average electric field.

The resistivity calculation module 122 may calculate a reciprocal of the effective electrical conductivity of the electrode structure as the calculated resistivity of the electrode structure.

The correction module 130 may calculate a deviation by comparing the calculated resistivity with a target resistivity, and may correct a variable or shape of an electrode structure in a direction of minimizing the deviation. Here, the calculated resistivity means a resistivity value of the electrode structure calculated by the resistivity calculation module 122, and the correction module 130 may receive the calculated resistivity from the resistivity calculation module 122. The target resistivity means a resistivity value of the electrode structure to be designed through the three-dimensional electrode structure design system 10, and may be a value input from the outside such as a user terminal or a higher-level controller. Variables of the electrode structure may include an intrinsic electrical conductivity of the active material, an intrinsic electrical conductivity of CBD particles, or an intrinsic electrical conductivity of the current collector. The shape of the electrode structure may include a degree of dispersion of CBD particles in a domain of the electrode structure.

According to an embodiment, the correction module 130 may calculate the deviation between the calculated resistivity and the target resistivity, may perform correction for the electrode structure when the deviation between the calculated resistivity and the target resistivity is greater than or equal to a predetermined reference value, and may not perform correction for the electrode structure when the deviation between the calculated resistivity and the target resistivity is less than the predetermined reference value.

The correction module 130 may correct a variable of the electrode structure using a variable tracking algorithm. According to an embodiment, the correction module 130 may compare magnitudes of the target resistivity and the calculated resistivity, and correct the electrode structure by correcting at least one of the intrinsic electrical conductivity of the active material, the intrinsic electrical conductivity of CBD particles, or the intrinsic electrical conductivity of the current collector. For example, the correction module 130 may decrease the intrinsic electrical conductivity of the active material, the intrinsic electrical conductivity of CBD particles, or the intrinsic electrical conductivity of the current collector when the target resistivity is larger than the calculated resistivity, and may increase the intrinsic electrical conductivity of the active material, the intrinsic electrical conductivity of CBD particles, or the intrinsic electrical conductivity of the current collector when the target resistivity is smaller than the calculated resistivity.

The correction module 130 may correct the shape of the electrode structure using a shape tracking algorithm. According to an embodiment, the correction module 130 may compare magnitudes of the target resistivity and the calculated resistivity, and correct the shape of the electrode structure by correcting the degree of dispersion of CBD particles in a domain of the electrode structure. For example, the correction module 130 may agglomerate CBD particles in the domain of the electrode structure when the target resistivity is larger than the calculated resistivity, and may disperse CBD particles in the domain of the electrode structure when the target resistivity is smaller than the calculated resistivity.

According to an embodiment, the correction module 130 may additionally form CBD particles in a domain of the electrode structure when a volume deviation between CBD particles in the corrected electrode structure and CBD particles in the electrode structure before correction is greater than or equal to a predetermined reference value. At this time, the corrected electrode structure may be one in which the degree of dispersion of CBD particles in a domain of the electrode structure is corrected through the shape tracking algorithm. Through this, even if the dispersion degree of CBD particles in the electrode structure is corrected, the volume of the CBD particles in the electrode structure may be adjusted to be constant.

The correction module 130 may calculate the calculated resistivity of the corrected electrode structure, and determine whether it is necessary to repeat the correction for the electrode structure based on whether a deviation between the calculated resistivity of the corrected electrode structure and the target resistivity is greater than or equal to a predetermined value. For example, the correction module 130 may repeat the correction for the electrode structure when the deviation between the calculated resistivity of the corrected electrode structure and the target resistivity is greater than or equal to the predetermined reference value, and may not repeat the correction for the electrode structure when the deviation between the calculated resistivity of the corrected electrode structure and the target resistivity is less than the predetermined reference value.

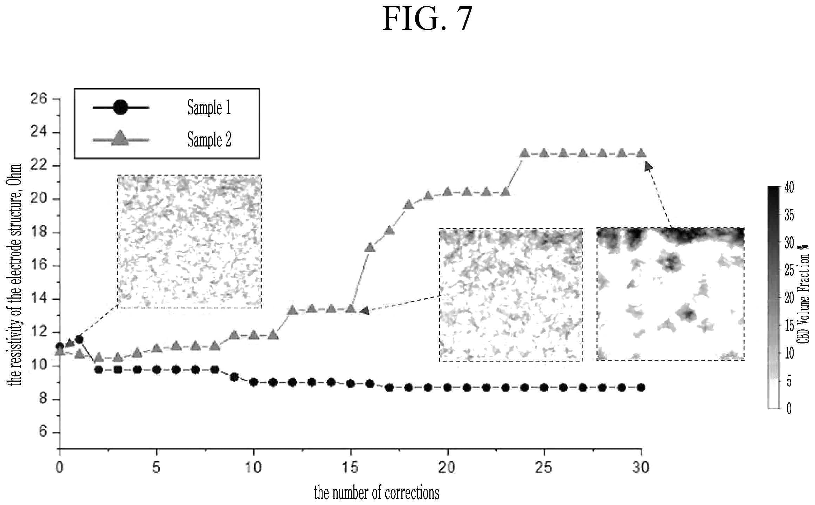

FIG. 7 shows a process in which the correction module corrects the shape of the electrode structure using the shape tracking algorithm according to an embodiment of the present invention.

Sample 1 of FIG. 7 shows a process in which the shape of the electrode structure is corrected when the target resistivity is 5 Ohm, and Sample 2 of FIG. 7 shows a process in which the shape of the electrode structure is corrected when the target resistivity is 25 Ohm. Referring to FIG. 7, it may be seen that in the case of Sample 1, the resistivity of the electrode structure decreases as the number of corrections increases, and in the case of Sample 2, the resistivity of the electrode structure increases as the number of corrections increases.

The backtracking module 140 may receive the corrected electrode structure from the correction module 130. The backtracking module 140 may calculate the intrinsic electrical conductivity of the active material, the intrinsic electrical conductivity of CBD particles, the intrinsic electrical conductivity of the current collector, and a third design parameter included in the corrected electrode structure. At this time, the intrinsic electrical conductivity of the active material, the intrinsic electrical conductivity of CBD particles, the intrinsic electrical conductivity of the current collector, and the third design parameter included in the corrected electrode structure may be values adjusted by the correction module 130. In calculating the intrinsic electrical conductivity of the active material, the intrinsic electrical conductivity of CBD particles, the intrinsic electrical conductivity of the current collector, and the third design parameter included in the corrected electrode structure, the backtracking module 140 may apply various conventionally known methods.

Here, the third design parameter means a variable for the corrected electrode structure, and may include content and true density of each component included in the electrode structure, size distribution and sphericity of active material, content ratio of conductive additive to binder, CBD dispersion degree, current collector thickness, and the like.

The backtracking module 140 may provide the intrinsic electrical conductivity of the active material, the intrinsic electrical conductivity of CBD particles, the intrinsic electrical conductivity of the current collector, and the third design parameter obtained from the corrected electrode structure to a user through a user terminal, a higher-level controller, or the like.

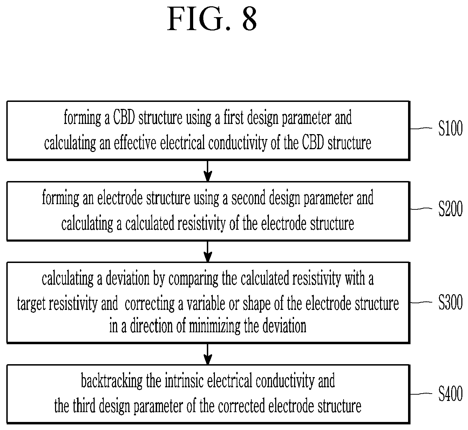

FIG. 8 is a flowchart of the three-dimensional electrode structure design method according to an embodiment of the present invention.

Referring to FIG. 8, the three-dimensional electrode structure design method according to an embodiment of the present invention may comprise a conductive additive and binder structure forming step S100, an electrode structure forming step S200, a step of correcting the electrode structure S300, and a backtracking step S400.

In the conductive additive and binder structure forming step S100, a structure forming module 110 may form a CBD structure using a first design parameter input for a CBD structure, and a calculation module 120 may calculate an effective electrical conductivity of the CBD structure using an intrinsic electrical conductivity of a conductive additive and an intrinsic electrical conductivity of a binder.

According to an embodiment, the conductive additive and binder structure forming step S100 may comprise setting, by the structure forming module 110, the domain and voxel sizes based on the first design parameter and forming a conductive additive in a domain using the first design parameter.

In the electrode structure forming step S200, the structure forming module 110 may form an electrode structure using a second design parameter input for the electrode structure, and the calculation module 120 may calculate an effective electrical conductivity of the electrode structure using an intrinsic electrical conductivity of the active material, an intrinsic electrical conductivity of CBD particles, and an intrinsic electrical conductivity of the current collector, and calculate the calculated resistivity of the electrode structure by converting the effective electrical conductivity of the electrode structure into a reciprocal.

According to an embodiment, the electrode structure forming step S200 may comprise setting, by the structure forming module 110, the domain and voxel sizes based on the second design parameter and forming an active material, CBD particles, and a current collector in a domain using the second design parameter.

According to an embodiment, the electrode structure forming step S200 may comprise calculating, by the calculation module 120, the effective electrical conductivity of the electrode structure by substituting the effective electrical conductivity of the CBD structure for the intrinsic electrical conductivity of CBD particles.

In the step of correcting the electrode structure S300, a correction module 130 may calculate a deviation by comparing the calculated resistivity with a target resistivity, and correct a variable or shape of an electrode structure in a direction of minimizing the deviation.

According to an embodiment, the step of correcting the electrode structure S300 may comprise: calculating, by the correction module 130, a deviation between the calculated resistivity and the target resistivity; and performing correction for the electrode structure when the deviation between the calculated resistivity and the target resistivity is greater than or equal to a predetermined reference value, and not performing correction for the electrode structure when the deviation between the calculated resistivity and the target resistivity is less than the predetermined reference value.

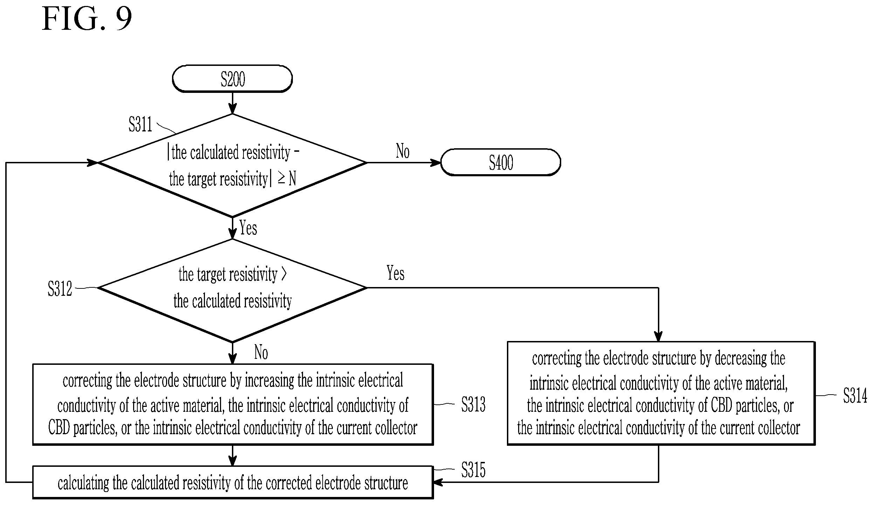

FIG. 9 is a flowchart of a step of correcting the electrode structure S300 according to an embodiment of the present invention.

Referring to FIG. 9, in the step of correcting the electrode structure S300 according to an embodiment of the present invention, the correction module 130 may correct a variable of the electrode structure using a variable tracking algorithm.

According to an embodiment, the step of correcting the electrode structure S300 may comprise calculating a deviation between the calculated resistivity and the target resistivity and determining whether the deviation between the calculated resistivity and the target resistivity is greater than or equal to a predetermined reference value S311, comparing magnitudes of the calculated resistivity and the target resistivity when the deviation between the calculated resistivity and the target resistivity is greater than or equal to the predetermined reference value S312, correcting the electrode structure by increasing the intrinsic electrical conductivity of the active material, the intrinsic electrical conductivity of CBD particles, or the intrinsic electrical conductivity of the current collector when the target resistivity is smaller than the calculated resistivity S313, correcting the electrode structure by decreasing the intrinsic electrical conductivity of the active material, the intrinsic electrical conductivity of CBD particles, or the intrinsic electrical conductivity of the current collector when the target resistivity is larger than the calculated resistivity S314, and calculating the calculated resistivity of the corrected electrode structure S315.

Meanwhile, in S311, correction for the electrode structure may not be performed when the deviation between the calculated resistivity and the target resistivity is less than the predetermined reference value. That is, in the step of correcting the electrode structure S300, the correction module 130 may repeatedly perform correction for a variable of the electrode structure until the deviation between the calculated resistivity and the target resistivity is less than the predetermined reference value.

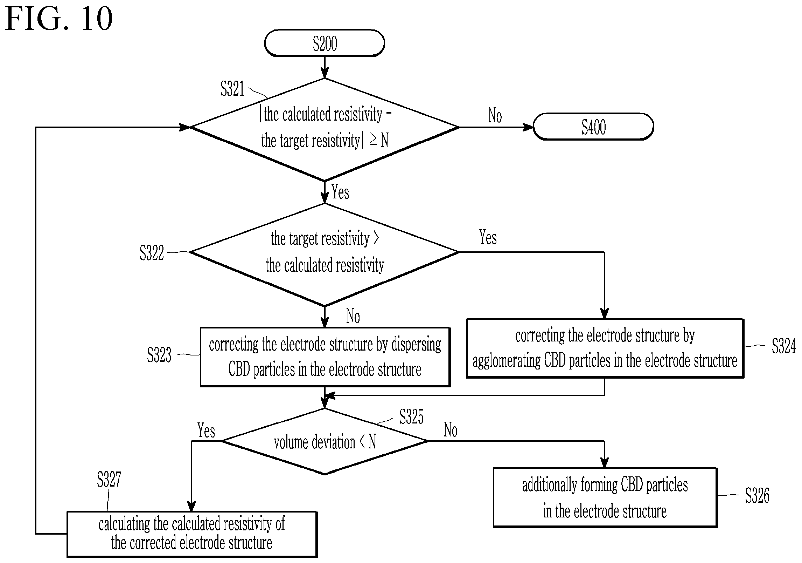

FIG. 10 is a flowchart of the step of correcting the electrode structure S300 according to another embodiment of the present invention.

Referring to FIG. 10, in the step of correcting the electrode structure S300 according to an embodiment of the present invention, the correction module 130 may correct the shape of the electrode structure using a shape tracking algorithm.

According to an embodiment, the step of correcting the electrode structure s300 may comprise calculating a deviation between the calculated resistivity and the target resistivity and determining whether the deviation between the calculated resistivity and the target resistivity is greater than or equal to a predetermined reference value S321, comparing magnitudes of the calculated resistivity and the target resistivity when the deviation between the calculated resistivity and the target resistivity is greater than or equal to the predetermined reference value S322, correcting the electrode structure by dispersing CBD particles in the domain of the electrode structure when the target resistivity is smaller than the calculated resistivity S323, correcting the electrode structure by agglomerating CBD particles in the domain of the electrode structure when the target resistivity is larger than the calculated resistivity S324, determining whether a volume deviation between CBD particles in the corrected electrode structure and CBD particles in the electrode structure before correction is less than a predetermined reference value S325, additionally forming CBD particles in the domain of the electrode structure when the volume deviation between the CBD particles in the corrected electrode structure and the CBD particles in the electrode structure before correction is greater than or equal to the predetermined reference value S326, and calculating the calculated resistivity of the corrected electrode structure when the volume deviation between the CBD particles in the corrected electrode structure and the CBD particles in the electrode structure before correction is less than the predetermined reference value S327.

Meanwhile, in S321, correction for the electrode structure may not be performed when the deviation between the calculated resistivity and the target resistivity is less than the predetermined reference value. That is, in the step of correcting the electrode structure S300, the correction module 130 may repeatedly perform correction for the shape of the electrode structure until the deviation between the calculated resistivity and the target resistivity is less than the predetermined reference value.

In the parameter backtracking step S400, the backtracking module 140 may calculate the intrinsic electrical conductivity of the active material, the intrinsic electrical conductivity of CBD particles, the intrinsic electrical conductivity of the current collector, and the third design parameter included in the corrected electrode structure.

Meanwhile, the above-described method may be written as a program that may be executed on a computer, and may be implemented on a general-purpose digital computer that operates the program using a computer-readable recording medium. The computer-readable recording medium may include a storage medium such as magnetic storage media such as ROM, RAM, USB, floppy disk, hard disk, or optical reading media such as CD-ROM and DVD.

The scope of the present invention is defined by the following patent claims rather than the above detailed description, and it should be interpreted that all changes or modified forms derived from the meaning and scope of the patent claims and their equivalent concepts are included in the scope of the present invention.

## Claims

1. A three-dimensional electrode structure design system, comprising:
a structure forming module configured to form a conductive additive and binder (CBD) structure using a first design parameter input for the CBD structure, and form an electrode structure using a second design parameter input for the electrode structure;
a calculation module configured to calculate an effective electrical conductivity of the CBD structure using an intrinsic electrical conductivity of a conductive additive and an intrinsic electrical conductivity of a binder, calculate an effective electrical conductivity of the electrode structure using an intrinsic electrical conductivity of the active material, an intrinsic electrical conductivity of CBD particles, and an intrinsic electrical conductivity of the current collector, and calculate a calculated resistivity of the electrode structure by converting the effective electrical conductivity of the electrode structure into a reciprocal;
a correction module configured to calculate a deviation by comparing the calculated resistivity with a target resistivity and correct a variable or shape of an electrode structure in a direction of minimizing the deviation; and
a backtracking module configured to calculate the intrinsic electrical conductivity of the active material, the intrinsic electrical conductivity of CBD particles, the intrinsic electrical conductivity of the current collector, and a third design parameter, all of which are included in the corrected electrode structure.

2. The three-dimensional electrode structure design system according to claim 1,
wherein the structure forming module comprises the conductive additive and binder structure forming module configured to set the domain and voxel sizes based on the first design parameter and form the conductive additive in the domain using the first design parameter.

3. The three-dimensional electrode structure design system according to claim 1,
wherein the structure forming module comprises an electrode structure formation module configured to set the domain and voxel sizes based on the second design parameter and form the active material, CBD particles, and the current collector in the domain using the second design parameter.

4. The three-dimensional electrode structure design system according to claim 1,
wherein the calculation module comprises an effective electrical conductivity calculation module configured to calculate the effective electrical conductivity of the CBD structure based on the Laplace equation and the Ohm's Law.

5. The three-dimensional electrode structure design system according to claim 1,
wherein the calculation module comprises a resistivity calculation module configured to calculate the effective electrical conductivity of the electrode structure by substituting the effective electrical conductivity of the CBD structure for the intrinsic electrical conductivity of CBD particles.

6. The three-dimensional electrode structure design system according to claim 1,
wherein the correction module,
calculates a deviation between the calculated resistivity and the target resistivity, performs correction for the electrode structure when the deviation is greater than or equal to a predetermined reference value, and does not perform correction for the electrode structure when the deviation is less than the predetermined reference value.

7. The three-dimensional electrode structure design system according to claim 1,
wherein the correction module,
when a deviation between the calculated resistivity and the target resistivity is greater than or equal to a predetermined reference value, compares magnitudes of the calculated resistivity and the target resistivity, and
when the target resistivity is smaller than the calculated resistivity, corrects the electrode structure by increasing the intrinsic electrical conductivity of the active material, the intrinsic electrical conductivity of CBD particles, or the intrinsic electrical conductivity of the current collector.

8. The three-dimensional electrode structure design system according to claim 1,
wherein the correction module,
when a deviation between the calculated resistivity and the target resistivity is greater than or equal to a predetermined reference value, compares magnitudes of the calculated resistivity and the target resistivity, and
when the target resistivity is larger than the calculated resistivity, corrects the electrode structure by decreasing the intrinsic electrical conductivity of the active material, the intrinsic electrical conductivity of CBD particles, or the intrinsic electrical conductivity of the current collector.

9. The three-dimensional electrode structure design system according to claim 1,
wherein the correction module,
when a deviation between the calculated resistivity and the target resistivity is greater than or equal to a predetermined reference value, compares magnitudes of the calculated resistivity and the target resistivity, and
when the target resistivity is smaller than the calculated resistivity, corrects the electrode structure by dispersing CBD particles in the domain of the electrode structure.

10. The three-dimensional electrode structure design system according to claim 1,
wherein the correction module,
when a deviation between the calculated resistivity and the target resistivity is greater than or equal to a predetermined reference value, compares magnitudes of the calculated resistivity and the target resistivity, and
when the target resistivity is larger than the calculated resistivity, corrects the electrode structure by agglomerating CBD particles in the domain of the electrode structure.

11. The three-dimensional electrode structure design system according to claim 1,
wherein the correction module,
additionally forms CBD particles in a domain of the electrode structure when a volume deviation between CBD particles in the corrected electrode structure and CBD particles in the electrode structure before the shape is corrected is greater than or equal to a predetermined reference value.

12. The three-dimensional electrode structure design system according to claim 1,
wherein the correction module,
calculates the calculated resistivity of the corrected electrode structure, and determines whether it is necessary to repeat correction for the electrode structure based on whether a deviation between the calculated resistivity of the corrected electrode structure and the target resistivity is greater than or equal to a predetermined value.

13. A three-dimensional electrode structure design method, comprising:
forming, by a structure forming module, a conductive additive and binder (CBD) structure using a first design parameter input for the CBD structure, and calculating, by a calculation module, an effective electrical conductivity of the CBD structure using an intrinsic electrical conductivity of a conductive additive and an intrinsic electrical conductivity of a binder;
forming, by the structure forming module, an electrode structure using a second design parameter input for the electrode structure, calculating, by the calculation module, an effective electrical conductivity of the electrode structure using an intrinsic electrical conductivity of the active material, an intrinsic electrical conductivity of CBD particles, and an intrinsic electrical conductivity of the current collector, and calculating a calculated resistivity of the electrode structure by converting the effective electrical conductivity of the electrode structure into a reciprocal;
calculating, by a correction module, a deviation by comparing the calculated resistivity with a target resistivity, and correcting a variable or shape of the electrode structure in a direction of minimizing the deviation; and
calculating, by a backtracking module, the intrinsic electrical conductivity of the active material, the intrinsic electrical conductivity of CBD particles, the intrinsic electrical conductivity of the current collector, and a third design parameter, all of which are included in the corrected electrode structure.

14. The three-dimensional electrode structure design method according to claim 13, wherein the step of calculating the effective electrical conductivity of the CBD structure comprises:
setting, by the structure forming module, the domain and voxel sizes based on the first design parameter; and
forming a conductive additive in the domain using the first design parameter.

15. The three-dimensional electrode structure design method according to claim 13, wherein the step of calculating the calculated resistivity of the electrode structure comprises:
setting, by the structure forming module, the domain and voxel sizes based on the second design parameter; and
forming an active material, CBD particles, and a current collector in a domain using the second design parameter.

16. The three-dimensional electrode structure design method according to claim 13, wherein the step of calculating the calculated resistivity of the electrode structure comprises:
calculating, by the calculation module, the effective electrical conductivity of the electrode structure by substituting the effective electrical conductivity of the CBD structure for the intrinsic electrical conductivity of CBD particles.

17. The three-dimensional electrode structure design method according to claim 13, wherein the step of correcting the variable or shape of an electrode structure comprises:
calculating, by the correction module, the deviation between the calculated resistivity and the target resistivity; and
performing correction for the electrode structure when the deviation between the calculated resistivity and the target resistivity is greater than or equal to a predetermined reference value, and not performing correction for the electrode structure when the deviation between the calculated resistivity and the target resistivity is less than the predetermined reference value.

18. The three-dimensional electrode structure design method according to claim 13, wherein the step of correcting the variable or shape of an electrode structure comprises:
calculating a deviation between the calculated resistivity and the target resistivity and determining whether the deviation between the calculated resistivity and the target resistivity is greater than or equal to a predetermined reference value;
when the deviation between the calculated resistivity and the target resistivity is greater than or equal to the predetermined reference value, comparing magnitudes of the calculated resistivity and the target resistivity;
when the target resistivity is smaller than the calculated resistivity, correcting the electrode structure by increasing the intrinsic electrical conductivity of the active material, the intrinsic electrical conductivity of CBD particles, or the intrinsic electrical conductivity of the current collector;
when the target resistivity is larger than the calculated resistivity, correcting the electrode structure by decreasing the intrinsic electrical conductivity of the active material, the intrinsic electrical conductivity of CBD particles, or the intrinsic electrical conductivity of the current collector; and
calculating the calculated resistivity of the corrected electrode structure.

19. The three-dimensional electrode structure design method according to claim 13, wherein the step of correcting the variable or shape of an electrode structure comprises:
calculating a deviation between the calculated resistivity and the target resistivity and determining whether the deviation between the calculated resistivity and the target resistivity is greater than or equal to a predetermined reference value;
when the deviation between the calculated resistivity and the target resistivity is greater than or equal to the predetermined reference value, comparing magnitudes of the calculated resistivity and the target resistivity;
when the target resistivity is smaller than the calculated resistivity, correcting the electrode structure by dispersing CBD particles in the domain of the electrode structure; and
when the target resistivity is larger than the calculated resistivity, correcting the electrode structure by agglomerating CBD particles in the domain of the electrode structure.

20. The three-dimensional electrode structure design method according to claim 19, wherein the step of correcting the variable or shape of an electrode structure comprises:
determining whether a volume deviation between CBD particles in the corrected electrode structure and CBD particles in the electrode structure before correction is less than a predetermined reference value;
additionally forming CBD particles in the domain of the electrode structure when the volume deviation between the CBD particles in the corrected electrode structure and the CBD particles in the electrode structure before correction is greater than or equal to the predetermined reference value; and
calculating the calculated resistivity of the corrected electrode structure when the volume deviation between the CBD particles in the corrected electrode structure and the CBD particles in the electrode structure before correction is less than the predetermined reference value.
